# EUROPEAN PATENT APPLICATION

(11) **EP 3 470 096 A1**
(43) Date of publication of application: **17.04.2019**
(21) Application number: 17196460.4
(22) Date of filing: 13.10.2017
(51) Int. Cl.: A61L 27/36, A61L 27/38, A61L 27/56

(54) **ELASTIN REDUCTION ALLOWING RECELLULARIZATION OF CARTILAGE IMPLANTS**

(71) Applicant: Erasmus University Medical Center Rotterdam, 3015 GE Rotterdam (NL); Medical University of Vienna, 1090 Vienna (AT)
(72) Inventor: van Osch, Geertruda Johanna Victoria Maria, 3015 GE Rotterdam (NL); Nuernberger, Sylvia, 1130 Vienna (AT); Lehmann, Johannes, 3015 GE Rotterdam (NL)
(74) Representative: V.O.

(57) **Abstract**

The invention relates to a method of producing an elastin-reduced cartilage scaffold, the method comprising the steps of providing an elastic cartilage sample and reducing elastin from said cartilage sample to produce an elastin-reduced cartilage scaffold. The invention further relates to an elastin-reduced cartilage scaffold. Said elastin-reduced cartilage scaffold can be used in a method of implantation *in vivo,* for repairment of cartilage, repairment of osteochondral defects and repairment of bone defects.

## Description

### Field of the invention

The present invention relates to the field of tissue implants. More specifically the invention is in the field of improvement of cartilage grafts. In particular, the invention relates to methods for reducing elastin of a cartilage scaffold that allows for recellularization of the scaffold.

### State of the art

Organs in the body may fail due to congenital disorders, acquired diseases, accidents and old age. Transplantation of organs is rendered difficult by both the scarcity of donor organs and the necessity to suppress the immune system to prevent organ rejection. In the last decades, our knowledge of cells that constitute an organ has rapidly expanded, rendering the creation of organs for transplantation from scratch, so called tissue engineering, increasingly realistic. Organs are a combination of cells and matrices, the latter providing a 3-dimensional structure that holds the cells in place. Cells carry markers unique for each person, allowing the body to recognize foreign tissue and reject it by elucidating an immune response, necessitating immune suppression after organ transplantation between different individuals. The molecules forming the matrices, however, are similar if not identical between people, and some molecules even between humans and other animals. Since matrices provide the structure for tissues, their use as a scaffold as a start to engineer organs seems attractive. For this, in a first step termed decellularization, cells are removed from an organ, leaving behind the scaffold. In a second step termed recellularization, cells derived from a recipient, i.e. a patient in need of the organ, may be infused to recolonize the scaffold. For organs such as heart and bladder, this approach has shown promise, although tissue engineering is still more a discipline of the bench than the bedside.

Cartilage allows rigid structures to be flexible. Cartilage is sturdier than, for example, muscle, yet capable of deforming without breaking in contrast to bone. Therefore, cartilage is present wherever flexibility is required. In windpipe and nose, it is sufficient stiff to keep the airway open, but also capable of deforming to accommodate the movements that are required for breathing and speaking. Its flexibility also allows cartilage to function as a shock absorber, quite like a bike tire, for example when present as a thin layer on bone surfaces in joints, or as intervertebral discs in the spine. The flexibility of cartilage is due to a simple principle: reversible water-binding in combination with a firm, yet elastic matrix. The cartilage scaffold is a network of structural fibers with water-binding molecules interlaced. When force is applied, the water is squeezed out of the cartilage and the tissue shrinks. When the force is removed, the water returns and the cartilage swells up to its original size.

Unlike most other tissues, cartilage does not heal. When damaged, for example after a trauma or during an operation, cartilage disintegrates, whereby the mechanical forces that are normally absorbed by the cartilage often accelerate the decay. Cartilage injuries are major healthcare problems - foremost in the medical disciplines of orthopaedics and head and neck surgery. Because the body is incapable of repairing damaged cartilage, the tissue must be replaced. Cartilage was one of the earliest tissues scientist tried to engineer because it contains only one cell type, chondrocytes, theoretically rendering it less of a challenge to engineer when compared to more complex organs such as the heart.

While many protocols have been developed to decellularize cartilage, step two, recellularization has proven impossible. Cartilage is not amendable to cell invasion, owing to its dense network of collagen fibers and scarcity of blood vessels. While chondrocytes residing in a cartilage scaffold can be removed when broken into pieces small enough to be washed out through the pores in the dense collagen network, new cells in general are too large to enter the network. In addition, cell adhesion to the extracellular matrix may be prevented by the presence of glycosaminoglycans (GAGs) (Bara et al., 2012. Connect Tissue Res 53: 220-8). Unpublished observations have indicated that chondrocytes, bone marrow-derived stromal cells (BMSCs) and even highly invasive cancer cell lines were incapable of migrating into decellularized cartilage. Moreover, decellularized native cartilage implanted under the skin of mice, a highly vascularized environment where the implant is prone to invasion by immune cells, did not show any signs of cell invasion (unpublished data).

Although existing methods for decellularization of a cartilage sample do not allow cells to invade and recellularize the cartilage scaffold, while maintaining structure and mechanical properties, recellularization of the cartilage scaffold is essential to assure fully functional cartilage, maintain the matrix and prevent degradation of the scaffold after transplantation. Achieving successful recellularization of the cartilage scaffold would therefore greatly improve the quality of a cartilage graft.

### Brief description of the invention

The invention provides a method of producing an elastin-reduced cartilage scaffold comprising the steps of a) providing an elastic cartilage sample and b) reducing of elastin from said cartilage sample to produce an elastin-reduced cartilage scaffold. In contrast to hyaline and fibrous cartilage, elastic cartilage contains large fiber bundles of elastin, which traverse the collagen network and surround the pockets, termed lacunae, in which the chondrocytes resides. The present invention solves the problem by removing elastin fibers from an elastic cartilage sample enabling cells to invade and attach to the elastin-reduced elastic cartilage scaffold.

The cartilage scaffold may be obtained from a human, for example from a human suffering from cartilage defects that will receive the cartilage scaffold as an implant, an allogeneic donor, i.e. not from a recipient of the implant, or from a xenogenic source. The resulting elastin-reduced cartilage scaffold consists mainly of proteins and fibers that are conserved in mammalian species. Hence, a scaffold from an allogeneic source or a xenogenic source is not expected to raise an immune reaction after implantation into a human recipient.

In methods of the invention, elastin fibers are reduced from the cartilage scaffold to about 50%, preferably about 25%, preferably about 10%, preferably 1%, preferably a complete removal, of the amount of elastin fibers prior to the step of reducing elastin fibers from the cartilage scaffold. This reduction preferably is obtained by incubating the cartilage scaffold with one or more enzymes, preferably comprising an elastase such as porcine pancreatic elastase. For this, the cartilage scaffold may be incubated with 1-12 U/ml elastase, preferably about 3 U/ml. Alternatively, the cartilage scaffold may be incubated with 0.01-1 U/ml elastase in combination with at least one other proteolytic enzyme, preferably pepsin, preferable about 0.5-5 mg/ml pepsin, preferably about 1 mg/ml pepsin.
Said incubation with one or more enzymes, preferably comprising an elastase, preferably comprises incubation of the cartilage scaffold for a period of 1-80 hours, preferably at least about 24-48 hours, preferably about 24 hours, to remove elastin.

Optionally, the cartilage sample is subjected to a decellularization step prior to reduction of elastin.

In case the cartilage sample is not sterile, the cartilage sample may be subjected to a decontamination step, prior to or after reduction of elastin.

Elastin-reduced elastic cartilage matrices can be used for repair of elastic cartilage defects, such as ear defects or ear malformations. However, it was surprisingly found that elastin-reduced elastic cartilage can also be used for the repair of hyaline cartilage defects, such as articular defects, nasal defects, airway defects and rib defects, for the repair of fibrous cartilage defects, such as intervertebral disc defects, and even for the repair of osteochondral defects and bone defects.

Recellularization of the cartilage scaffold may be accomplished by cells from a recipient after implantation, or by cells that are provided into the cartilage scaffold prior to implantation.

A method of the invention may comprise a step of recellularizing the elastin-reduced cartilage scaffold, by allowing cells to adhere into the elastin-reduced cartilage scaffold. This can be accomplished by seeding cells onto the elastin-reduced cartilage scaffold or by injecting cells into the elastin-reduced cartilage scaffold and allowing said cells to migrate into the empty channels and lacunae of the elastin-reduced cartilage scaffold. For injecting cells into the cartilage scaffold a needle may be used.

The cells used for recellularization of the cartilage scaffold are preferably cells with chondrogenic or osteogenic potential. Such cells include chondrocytes, perichondrium cells, periosteum cells and stem/progenitor cells, such as adipose-derived stromal vascular stem cells, synovium-derived mesenchymal cells and bone marrow-derived stromal cells. Said cells preferably are autologous human cells, i.e. cells from a recipient of the implant, or allogeneic human cells, i.e. cells from an individual that is not a recipient of the implant. Said allogeneic human cells are preferably genetically matched to the recipient of the implant, preferably by Human Leukocyte Antigen (HLA) matching and/or blood type matching.

The invention further provides an elastin-reduced cartilage scaffold as such, or a recellularized elastin-reduced cartilage scaffold, obtainable by the methods of the invention. Said cartilage scaffold can be used in a method of repairment of cartilage. Surprisingly, an elastin-reduced elastic cartilage scaffold can not only be used in a method of repairment of elastic cartilage, such as ear cartilage, but also for the repairment of hyaline cartilage and fibrous cartilage, such as articular, nasal and airway cartilage. Said cartilage scaffold can further be used in a method of repairment of an osteochondral defect and repairment of a bone defect.

### Brief description of the figures:

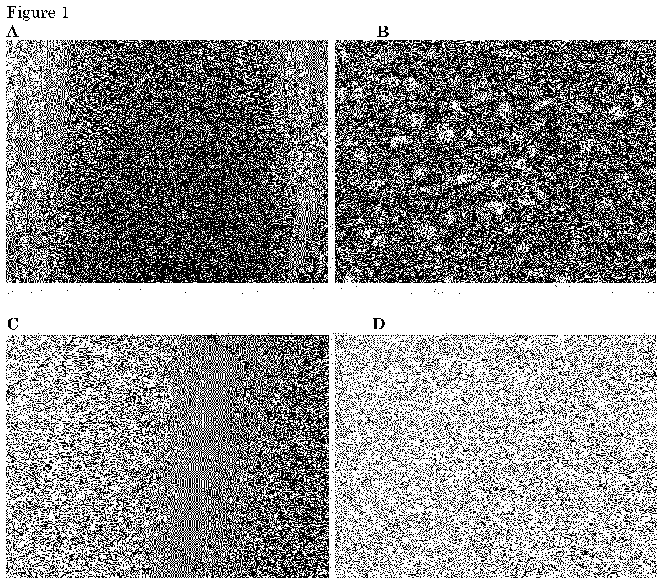

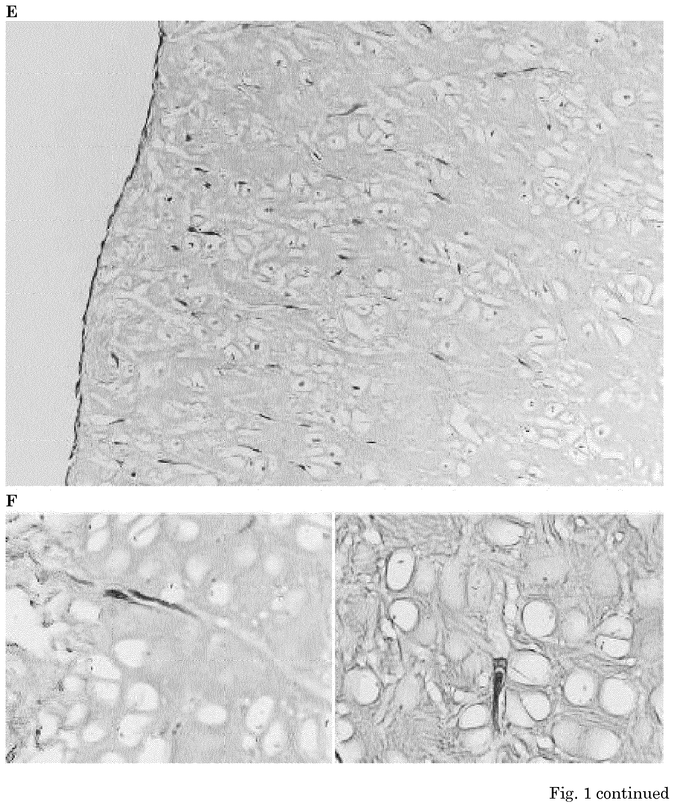

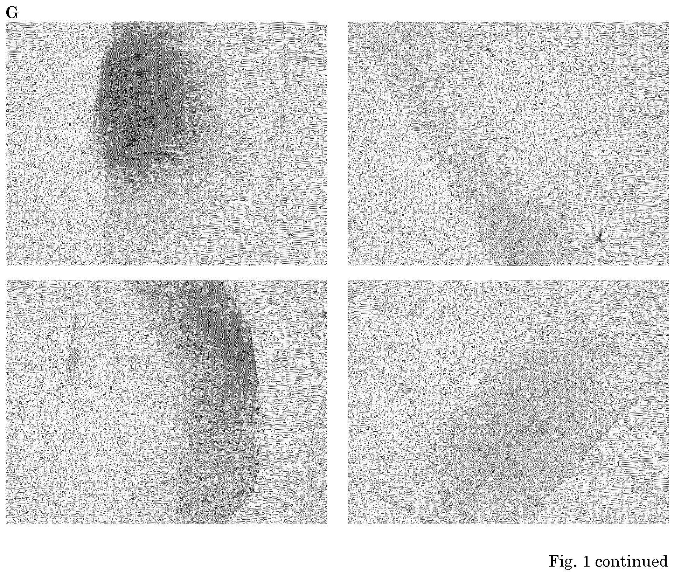

**Figure 1** (A-D) Rescorcin-fuchsin staining for elastic fiber bundles shows the presence of elastin fibers in bovine auricular cartilage (A, magnification in B) and removal of the fibers and opening of channels and lacunae after treatment with elastase (C, D). (E, F) Haematoxylin-Eosin staining of elastase-treated bovine auricular cartilage seeded with BMSCs after 6 days of culture (E) with cells migrating into the tissue, and (F, magnified) migrating through channels, displaying an elongated shape. (G) Thionin staining of elastin-reduced auricular cartilage reseeded with BMSCs and chondrogenically differentiated for 35 days. Dark staining shows glycosaminoglycans, indicating newly formed cartilage within the existing cartilage scaffold. Four different samples are displayed.

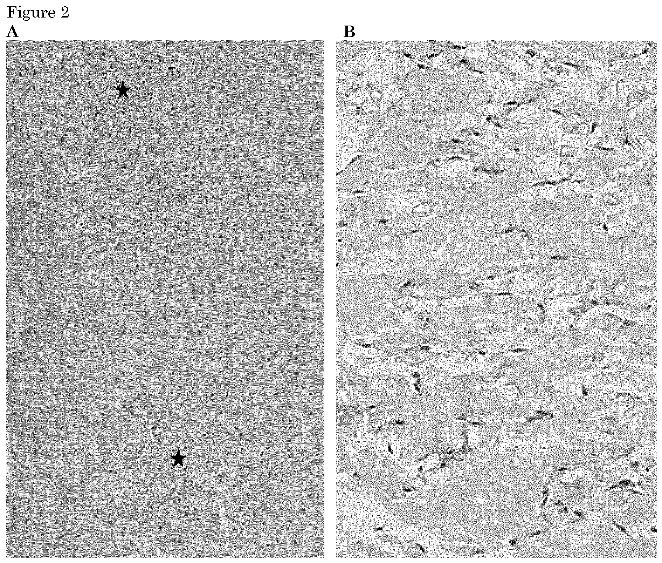

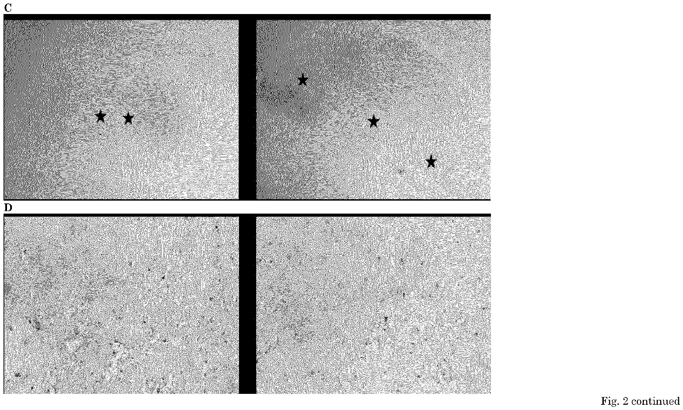

**Figure 2** (A, B) Haematoxylin-Eosin staining of elastin-reduced auricular cartilage injected with human articular chondrocytes and cultured for six days in chondrogenic medium shows (A) distribution of cells away from injection sites (indicated by black stars) and (B, magnified from A) a high cell density. (C, D) Thionin staining of elastin-reduced auricular cartilage injected with human articular chondrocytes and cultured for 35 days in chondrogenic medium shows *in situ* formation of a cartilage-like matrix, marked by dark colored areas, (C) away from the injection sites (indicated by black stars) and (D) around the migrating chondrocytes.

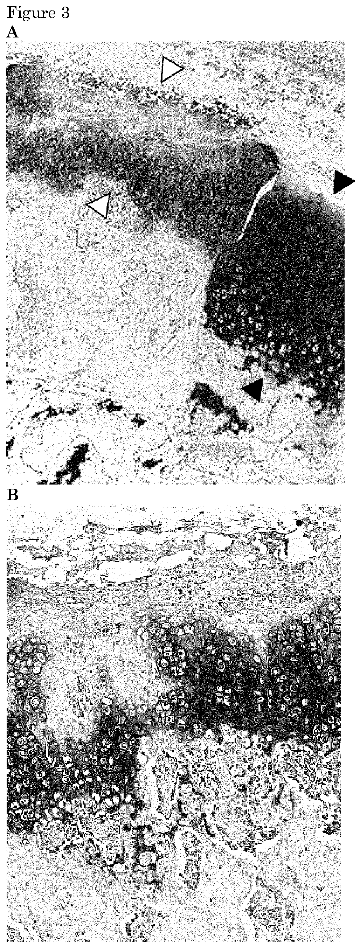

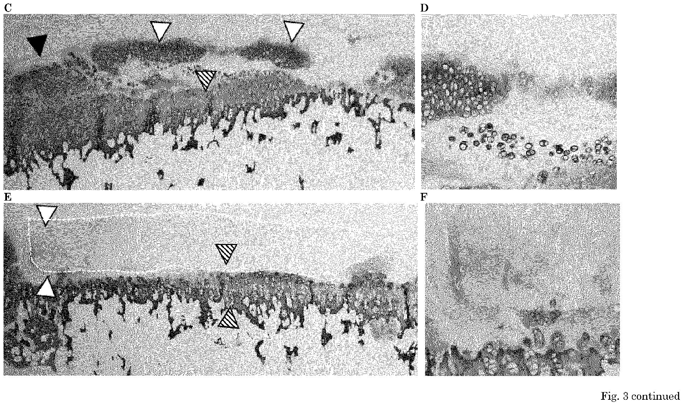

**Figure 3** (A-F) Elastin-reduced auricular cartilage placed into a bovine joint defect model implanted subcutaneously into a mouse. (A, B) Cartilage graft without seeded cells in osteochondral defects (A magnified in B). *In situ* formation of new cartilage, marked by dark colored areas after Thionin staining, due to invasion of cells from the bony part, and to a minor extent from the cartilage part, of the bovine joint defect model; white arrowheads indicate newly formed cartilage in elastin-reduced cartilage matrix; black arrowheads indicate native intact bovine cartilage. (C, magnified in D) When access to the bony part of the joint model was limited by an intact calcified cartilage layer below the defect (chondral defect), only cells from the cartilage part of the bovine joint defect model invaded the cartilage graft and formed new cartilage. White arrowheads indicate newly formed cartilage in elastin-reduced cartilage matrix; the black arrowhead indicates native intact bovine cartilage; the shaded arrowhead indicates native calcified bovine cartilage. (E, magnified in F) Elastin-reduced auricular cartilage, demarcated by white line, seeded with human articular chondrocytes, placed into a bovine joint defect model and implanted subcutaneously into a mouse. When the invasion of the cells from the bony part of the bovine joint defect model was prevented, cartilage-like matrix also forms within the auricular cartilage, indicating that seeded cells can revitalize the graft as well. White arrowheads indicate newly formed cartilage in elastin-reduced cartilage matrix; shaded arrowheads indicate native calcified bovine cartilage.

### Detailed description of the invention

### Definitions

The term 'decellularization', as used herein, refers to a process to remove cells and cell remnants from a cartilage extracellular matrix, leaving a cartilage scaffold of the original tissue.

The term 'recellularization', as used herein, refers to a process of allowing cells to invade into a cartilage scaffold following decellularization of said cartilage scaffold.

The term 'cartilage scaffold', as used herein, refers to the extracellular substance of cartilage composed predominantly of collagen fibers, non-collagenous glycoproteins and proteoglycans.

The term 'allogeneic', as used herein, refers to being derived from an individual that is not the recipient of the implant.

The term 'elastin', as used herein, refers to a highly elastic protein in cartilage tissue which allows cartilage tissue in the body to resume its shape after stretching or contracting.

The term 'elastin fibers', as used herein, refers to bundles of elastin protein found in extracellular scaffold of cartilage tissues.

The term 'elastase', as used herein, refers to a serine endopeptidase of the peptidase S1 family (see http://enzyme.expasy.org/peptidas.txt). A preferred elastase is an enzyme from a class with EC number 3.4.21.36, 3.4.21.37 or 3.4.21.71. An elastase hydrolyzes proteins, including elastin. One unit of elastase is defined as the amount of enzyme that will hydrolyze 1.0 µmole of N-succinyl-L-Ala-Ala-Ala-p-nitroanilide per minute, at pH 8 and 25°C.

The term 'elastic cartilage' refers to cartilage containing elastic fibers next to collagen fibers, such as present in the ear, Eustachian tubes and epiglottis.

The term 'hyaline cartilage' refers to cartilage containing mainly collagen type II fibers, such as cartilage of articulating, opposing surfaces of bones within synovial joints, ventral ends of ribs, larynx, trachea, bronchi, nasal septum and ala of the nose.

The term 'fibrous cartilage', as used herein, refers to cartilage containing collagen type I next to collagen type II fibers and is present in the meniscus, in the annulus fibrosus of intervertebral disks, the temporomandibular joint and the pubic symphysis.

The term 'auricular cartilage', as used herein, refers to cartilage of the ear's auricle, the outermost portion of an ear.

The term 'articular cartilage', as used herein, refers to cartilage covering articulating, opposing surfaces of bones within synovial joints.

The term 'perichondrium', as used herein, refers to a layer of dense irregular connective tissue that surrounds cartilage, except at the endings of joints and consists of two separate layers; an outer fibrous layer, containing collagen-producing fibroblasts, and an inner chondrogenic layer that contains progenitor cells that can form chondroblasts and chondrocytes.

The term 'chondroblast', as used herein, refers to an immature cartilage-producing cell.

The term 'chondrocytes', as used herein, refers to specialized cells which constitute and produce cartilage.

The term 'periosteum', as used herein, refers to a specialized connective tissue that covers all bones of a body except at joints. Periosteum has cartilage- and bone-forming potential.

The term 'bone marrow-derived stromal cells', or BMSCs, as used herein, refers to mesenchymal cells derived from bone marrow and identified by adherence to plastic and lack of expression of hematopoietic markers such as CD45 and CD14.

The term `synovium-derived mesenchymal cells', as used herein, refers to mesenchymal cells derived from synovium membrane tissue and identified by adherence to plastic. Said cells lack of expression of hematopoietic markers such as CD45 and CD14.

The term 'adipose-derived stromal vascular stem cells', as used herein, refers to mesenchymal progenitor/stem cells derived from the stromal vascular fraction from adipose tissue identified by lack of expression of hematopoietic markers such as CD45 and CD14.

The term 'chondrogenic potential', as used herein, refers to the ability of cells to produce cartilage.

The term 'osteogenic potential', as used herein, refers to the ability of cells to produce bone.

The term 'sonication', as used herein, refers to the act of applying sound energy to agitate particles in a sample.

The term 'disorder', as used herein, refers to a abnormality of function, structure, or both, resulting from genetic or embryonic failure in development or from exogenous factors such as trauma or disease.

The term 'treatment' or 'treating', as used herein, refers to clinical intervention in an attempt to alter the natural course of the individual being treated. Desirable effects of the treatment include preventing occurrence or recurrence of a disorder, alleviation of symptoms, diminishment of any direct or indirect consequences of a disorder, and/or amelioration or palliation of the state of a disorder. In the present invention, 'treatment' or 'treating' is understood to mean amelioration or palliation of a human suffering from a chondropathy such as damaged articular cartilage, or of a malformation or absence of cartilage tissue such as the nose or the external ear, by administering a composition comprising an cartilage scaffold with or without recellularized cells.

The term 'repairment', as used herein, refers to the action of in part or completely repairing a tissue, preferably a cartilage tissue.

### Provision of a cartalage scaffold

Cartilage is usually classified into three types based on histological appearance, a) hyaline cartilage, which can be derived from joints, ribs, nose or trachea, b) fibrous cartilage, which can be found in intervertebral disks, scar tissue and meniscus and c) elastic cartilage, which can be obtained from the epiglottis, outer ear and the Eustachian tubes. A preferred cartilage sample is an elastic cartilage sample.

Cartilage samples can be obtained from a donor, for example by removing surrounding tissue such as skin and excision, preferably employing methods that ensure that the resulting cartilage sample is sterile.

A cartilage sample may be obtained from an animal such as, but not limited to, cow or pig, and from a human. Human cartilage samples can be obtained, for example, from post mortem donors, or as surgical specimens resulting from a relevant procedure, such as during reshaping of the ear (otoplasty). Animal cartilage, for example from a cow or a pig, such as adult bovine (cow) and immature bovine (calf) cartilage, can be obtained from a slaughterhouse.

A cartilage sample is optionally decellularized. During this decellularization step, resident chondrocytes in the cartilage samples are killed and dislodged by subjecting the samples to at least one freeze-thaw cycle, preferably to at least three freeze-thaw cycles, such as 3 freeze-thaw cycles, 4 freeze-thaw cycles, 5 freeze-thaw cycles or 6 freeze-thaw cycles. A freeze-thaw cycle kills cells and helps removing cellular particles.

Said freeze-thaw cycle(s) may be performed dry or after incubation of the cartilage sample in a hypotonic buffered solution, for example in 10 mM Tris-HCl at pH=8, or in deionized water (ISO 3696 Grade I). A preferred method includes at least one dry freeze-thaw cycle and at least one freeze-thaw cycle after incubation of the cartilage sample in a hypotonic buffered solution, such as two dry freeze-thaw cycles, followed by two freeze-thaw cycles of the cartilage sample in a hypotonic buffered solution. Each of said freeze-thaw cycle may independently be performed at a temperature between -200 °C and + 40°C.

In case the cartilage sample is not sterile, said cartilage sample can be decontaminated prior to or after reduction of elastin, for example by incubation in a buffered aqueous solution comprising at least one of ethanol, peracetic acid, iodine solution, sodium hydroxide solution, hydrogen chloride solution and/or hydrogen peroxide, preferably hydrogen peroxide, for 0.2 - 10 hours, preferably 1-3 hours. For example, a decontamination step may be performed by incubating a cartilage sample in 0.1% peracetic acid in PBS for 3 hours at room temperature, as shown by Utomo et al., 2015 (Utomo et al., 2015. Biomed Mater 10: 015010). In a preferred decontamination step, a cartilage sample is decontaminated by incubation in 5% hydrogen peroxide for 60 minutes at 37°C.

### Reducing elastin in a cartalage scaffold

Especially elastic cartilage contains large fiber bundles of elastin, giving it superior flexibility. The elastin fibers traverse the collagen network and surround the pockets, termed lacunae, in which the chondrocytes resides. Removing these elastin fibers from a cartilage scaffold will leave behind empty channels traversing the collagen network.

Said elastin fibers are preferably reduced or even completely removed from a cartilage scaffold, preferably by a serine protease such as elastase, preferably a porcine pancreatic elastase. While other elastases could also be used for the reduction or removal of elastin from the cartilage scaffold, they may cause some damage to the collagen network. For reduction of elastin, the cartilage scaffold is preferably incubated with a serine protease such as elastase, preferably porcine pancreatic elastase, in a concentration of 1-12 U/ml, preferably 3 U/ml, for a period of 1-80 hours, preferably 24-48 hours. Said incubation time may in part be determined by the sample size and thickness. For example, the incubation time preferably is about 24 hours for samples smaller than 1 cm² with a thickness of 3 mm, and about 48 to 72 hours for samples larger than 1 cm². In Utomo et al., 2015 (Utomo et al., 2015. Biomed Mater 10: 015010), the samples were incubated with 0.03 U/ml elastase for 24 hours at 37°C. This procedure, however, did not noticeably modify the elastin content in the cartilage tissue to allow for recellularization. Said elastin concentration may be lower, preferably about 0.01-1 U/ml, if elastase treatment of a cartilage sample is combined with treatment with other proteolytic enzymes. These other proteolytic enzyme may comprise pepsin, preferably at a concentration of about 0.5-5 mg/ml pepsin, preferably at a concentration of about 1 mg/ml pepsin. All enzymes preferably have a purity of more than 90% and/or are produced according to Good Manufacturing Practice (GMP).

The samples may be subjected to, for example, 2x dry freeze-thaw cycles and 2x freeze-thaw cycles in hypotonic buffer, followed by overnight incubation in 1 mg/mL pepsin in 0.5 M acetic acid and overnight incubation with 0.03 U/ml elastase in Tris buffer pH 8.6, after which the samples are washed with PBS for 2x 30 min followed by an overnight wash. Said step of reducing or completely removing elastin from a cartilage scaffold, preferably is followed by incubating the cartilage scaffold in a nuclease solution to remove all nucleic acid material from the scaffold. For this, said scaffold may be incubated in a non-toxic buffered saline solution comprising 1-100 U/ml, preferably about 50 U/ml, of a DNase and/or 0.1-10 U/ml, preferably about 1 U/ml, of a RNase, followed by repeated washings in a non-toxic buffered saline solution in the presence or absence of a protease inhibitor such as a competitive serine protease inhibitor.

### Methods for recellularazataon of cartalage samples in vitro

After reduction or complete removal of elastin from a cartilage scaffold, the lacunae will be open and channels occur where elastin fibers traversed the matrix, facilitating removal of cell remnants. Said reduction or complete removal of elastin may also result in a reduction of glycosaminoglycans (GAGs) in the cartilage scaffold. Since it has been reported that adhesion of cells, such as chondrocytes, may be prevented by GAGs (Bara et al., 2012. Connect Tissue Res 53: 220-8), the reduction of GAGs in the cartilage scaffold may be beneficial for the recellularization of the scaffold. The channels and accessible lacunae now allow cells to invade the scaffold, permitting recellularization of cartilage.

Reduction or removal of elastin bundles gives way to channels in elastic cartilage such as the elastic cartilage in pinnae. In hyaline cartilage, which contains disperse elastin fibers, but lacks elastin bundles, reduction or removal of elastin does not give way to channels.

Cells that are suitable for recellularization of an elastin-reduced, cartilage scaffold are cells with chondrogenic potential such as chondrocytes, perichondrium cells, periosteum cells, and mesenchymal stem/progenitor cells such as adipose-derived stromal vascular stem cells, bone marrow-derived stromal cells or synovium derived mesenchymal cells.

Methods for culturing cells with chondrogenic potential are known to a person skilled in the art. For example, BMSCs cells may be cultured at a density of 2300 cells/cm² at 37 °C and 5% CO₂, in medium, termed here expansion medium, comprising MEM-α (Gibco, Carlsbad, USA), containing 10% heat-inactivated FCS (Lonza, Verviers, Belgium), 50 µg/mL gentamicin, 1.5 µg/ml fungizone, 25µg/ml L-ascorbic acid 2-phosphate and 1 ng/mL basic Fibroblast Growth Factor 2 (bFGF2; R&D Systems, Minneapolis, USA) as described in Johnstone et al., 1998 (Johnstone et al., 1998. Exp Cell Res 238: 265-72), or an adaptation of this method that can improve cell proliferation and/or chondrogenic capacity, such as for example described in Narcisi et al., 2015 (Narcisi et al., 2015. Stem Cell Reports 4: 459-7).

Said chondrogenic cells, preferably human cells, may be seeded into a cartilage scaffold using one of the following methods, which are known to a person skilled in the art:
a) Static seeding; a cartilage scaffold may be placed at the bottom of a culture well, which is then filled with cells (typically about 1.000 cells/mm³ scaffold to 10.000 cells/mm³) in expansion medium. The cells will settle down and attach to the cartilage scaffold.
b) Dynamic seeding; a cartilage scaffold may be placed in a closed tube filled with cells (typically about 5.000 cells/mm³ scaffold to about 20.000 cells/mm³) in expansion medium and incubated while rotating at 45° angle.
c) Injection; a cell suspension of about 1000 cells/mm³ scaffold to about 10.000 cells/mm³ may be injected into a cartilage scaffold with a needle.

Chondrogenic cells such as BMSCs, when seeded onto the surface of the cartilage scaffold by static seeding, will migrate into the scaffold and distribute therein. Culture medium used here might contain a serum gradient or a chemokine enhancing migration rate, or preferably, enhance chondrogenic differentiation, for example DMEM-high glucose GlutaMAX+ (GIBCO), 1:100 insulin, transferrin and selenous acid (ITS+, BD Biosciences), 40 µg/ml L-proline (Sigma-Aldrich), 1mM sodium pyruvate (GIBCO), 100 nM dexamethasone (Sigma-Aldrich), 10 ng/ml transforming growth factor β1 (TGF-β1, R&D Systems), 2 µM WNT inhibitor IWP2 (Sigma-Aldrich), 1.5 µg/ml fungizone (GIBCO) and 50 µg/ml gentamycin (GIBCO) as described in Narcisi et al., 2015 (Narcisi et al., 2015. Stem Cell Reports 4: 459-7). The migrating cells may not evenly distribute over the cartilage scaffold and may grow in dense spots within the scaffold. Alternatively, the cells can be injected into the cartilage graft and from the injection site distribute further during subsequent culture or after implantation *in vivo.* All cells are preferably cultured according to Good Manufacturing Practice (GMP).

A graft can be implanted immediately after cell seeding or after further cell culture to stimulate migration and cartilage formation.

When induced to undergo chondrogenic differentiation, the cells will form cartilage within the scaffold. Unlike a collagen scaffold, the elastin-reduced cartilage scaffold retains its shape during cell seeding and chondrogenic differentiation.

### Implantataon of a recellularized cartilage scaffold

A recellularized cartilage scaffold can be used for implantation into a host. The recellularized cartilage scaffold may be used for cartilage repair, such as articular, nasal, airway, meniscus and ear cartilage. The cartilage scaffold can be used to repair both congenital cartilage defects as well as damaged cartilage tissues. Because the cartilage scaffold preserves its size and shape after elastin reduction and recellularization, such scaffold may be used to reconstruct large cartilage defects of cartilage tissues that hold complex shapes, such as the external ear cartilage.

During transport the recellularized cartilage scaffold preferably is kept sterile and moist, preferably by using a sterile transport container. The cartilage tissue that is to be repaired may be reached by making an incision in the skin, exposing the cartilage tissue, or by making an opening in a joint. Just prior to implantation the recellularized cartilage scaffold is removed from the sterile transport container and, if needed, made fit to size, for example with scissors, according to the template. The recellularized scaffold can be implanted to replace a damaged or missing cartilage tissue or a part thereof. The recellularized cartilage scaffold can be inserted by press-fitting, stitching or fibrin gluing it at the borders of the cartilage defect, preferably using methods for sterile working. After achieving control of bleeding, the opening in the joint or incision may be closed.

### Implantataon of an elastin-reduced cartilage scaffold

Although for total ear reconstruction recellularization prior to implantation might be preferred, a cell-free approach might work for the treatment of articular cartilage defects, osteochondral defects or for nasal or auricular defects where perichondrium is still intact. This approach involves the implantation of an elastin-reduced, non-recellularized cartilage scaffold into a host. The implantation procedure of a non-recellularized cartilage scaffold will be similar to the procedure described herein above for a recellularized cartilage scaffold.

Recellularization of the elastin-reduced cartilage scaffold may be achieved *in vivo* by ingrowth of autologous host cells from the environment into the scaffold. This can be stimulated, e.g. by adding growth factors, chemokine or other cartilage repair stimulating agents such as platelet-rich plasma.

### Examples

### Example 1: Recellularization with BMSCs by Dynamic Seeding

The outer ears of 12-week-old calves were washed with water and the perichondrium, skin and muscle were removed using a scalpel till only the cartilage remained. Subsequently, biopsies of 6 mm diameter and about 1 mm height were obtained from the auricular cartilage using biopsy punches, hence each sample being about 30 mm³ in volume.

The following protocol was then employed for removal of the cells and elastin fibers, the volumes given applied to batches of approximately 20 samples unless specified otherwise. First, the samples were rinsed thrice briefly with 40 ml ISO 3696 Grade I deionized water following referred to as deionized water, to remove debris from the isolation, such as loose pieces of skin. If necessary, samples were frozen in 20 ml of deionized water at -20°C for storage. Then samples were sterilized with 20 ml of 5% hydrogen peroxide for 60 minutes at 37°C. Subsequently, the samples were rinsed thrice briefly with 40 ml deionized water and thrice freeze-thawed from -80°C to 37°C in deionized water for time sufficient to completely freeze and thaw the samples respectively. The elastin fibers bundles were digested by incubating samples with 3 U/ml elastase (Sigma-Aldrich) in a solution of 2M tris(hydroxymethyl)aminomethane (TRIS) HCl in water, set to a pH of 8.6, at 1 ml per sample at 37°C for 24 hours rotating at 30 RPM with the samples being constantly submerged during rotation. The elastase solution was subsequently washed away by three times incubating with 50 ml deionized water for 5 min each rotating at 30 RPM at RT. To facilitate opening up of the lacunae and increase removal of cell debris out of the cartilage matrix, samples were exposed to physical strain: a snap freeze-thaw cycle (from -190°C to 37°C), sonication twice for 8 minutes at 42 kHz (70 Watt waterbath sonicator) and vortexing thrice, in absence of liquid, for about 30 seconds at 2500 RPM.

Subsequently, sterility was tested and the samples were coated with serum by incubating the samples in 40 ml MEM-α (Gibco, Carlsbad, USA), containing 10% heat-inactivated FCS (Lonza, Verviers, Belgium) for 24 hours at 37°C, rotating at 30 RPM. Sterile samples were afterwards placed in 2 ml polypropylene microcentrifuge tubes, with each sample placed in one tube, and seeded with human adult bone marrow-derived stromal cells (BMSCs) at 250.000 cells per sample (approximately 8.500 cells/mm³) in 1.5 ml expansion medium (MEM-α (Gibco, Carlsbad, USA), containing 10% heat-inactivated FCS (Lonza, Verviers, Belgium), 50 µg/mL gentamicin (Invitrogen Life Technologies), 1.5 µg/ml fungizone (Invitrogen Life Technologies), 25 µg/ml L-ascorbic acid 2-phosphate and 1 ng/mL basic Fibroblast Growth Factor 2 (bFGF2; R&D Systems, Minneapolis, USA). The samples were then incubated with the cells in the microcentrifuge tubes for 24 h at 37°C rotating at 30 RPM at an 45° angle. Following seeding, samples were gently rinsed twice in expansion medium and transferred to 2 cm² wells (each sample occupying one well) and cultured either in 1 ml/well expansion medium for six days, with medium being refreshed at day two and day four, or cultured for 35 days, with medium renewal every three days in chondrogenic medium (DMEM-high glucose GlutaMAX+ (GIBCO), 1:100 insulin, transferrin and selenous acid (ITS+, BD Biosciences), 40 µg/ml L-proline (Sigma-Aldrich), 1 mM sodium pyruvate (GIBCO), 100 nM dexamethasone (Sigma-Aldrich), 10 ng/ml transforming growth factor β1 (TGF-β1, R&D Systems), 1.5 µg/ml fungizone (Invitrogen Life Technologies) and 50 µg/ml gentamycin (Invitrogen Life Technologies)).

After culture, samples were harvested carefully with tweezers, washed gently with phosphate buffered saline (PBS) and fixed in 3.7% formalin for 24 hours. Fixed samples were then embedded in agarose, dehydrated, embedded in paraffin and sectioned at 6 µm thickness and the sections placed on glass slides and subsequently dried, rehydrated, stained with Haematoxylin (Sigma-Aldrich) and 2% Eosin (Merck) or 0.4% Thionin solution (Sigma-Aldrich) or Resorcin-Fuchsin soluation, (RF, Klinipath, Duiven, the Netherlands) dehydrated, and sealed under a glass coverslip, according to the manufacturer's protocol and as described by Narcisi et al., 2015 (Narcisi et al., 2015, Stem Cell Reports 4: 459-7) and Utomo et al., 2015. (Utomo et al., 2015. Biomed Mater 10: 015010).

Micrographs (Figure 1A-D) show the elastase-treated decellularized cartilage, with channels and opened-up lacunae. BMSCs can then migrate into the tissue within seven days (Figure 1E) and it can be seen that cells adopted an elongated shape when migrating through the channels left by the removed elastin fibers (Figure 1F). When chondrogenically differentiated, the BMSCs formed new cartilage-like matrix within the tissue within the decellularized matrix (Figure 1G).

### Example 2: In Situ Chondrogenesis after Injection

Some areas of cartilage are difficult to reach for cells by migration, for example the cartilage of the outer ear is surrounded by a dense perichondrium layer. As shown by Utomo et al., 2015 (Utomo et al., 2015. Biomed Mater 10: 015010) an entire ear can be decellularized using elastase. The perichondrium is tightly attached to the cartilage and removal of it without damaging the cartilage structure is not feasible.

To test whether recellularization of an entire ear is feasible, bovine auricular cartilage samples without necessitating removal of the perichondrium were recellularized with chondrogenic cells by injection through the perichondrium.

The outer ears of 12-week-old calves were washed with water and skin and muscle were removed using a scalpel till only the cartilage with its intact perichondrium remained. Subsequently, square biopsies of 15 mm side length were obtained from the auricular cartilage using a scalpel with each sample being about 2-4 mm in height (including perichondrium) and thus about 450-900 mm³ in volume.

The following protocol was then employed for removal of the cells and elastin fibers, the volumes given applied to one sample. First, the samples were rinsed thrice briefly with 10 ml ISO 3696 Grade I deionized water, following referred to as deionized water, to remove debris from the isolation, such as loose pieces of skin. If necessary, samples were frozen in 5 ml of deionized water at -20°C for storage. Then samples were sterilized twice with each 10 ml of 5% hydrogen peroxide for 120 minutes at 37°C. Subsequently, the samples were rinsed thrice briefly with 10 ml deionized water and thrice freeze-thawed from -80°C to 37°C in deionized water for time sufficient to completely freeze and thaw the samples respectively. The elastin fibers bundles were digested by incubating samples with 12 U/ml elastase (GoldBio) in a solution of 2M tris(hydroxymethyl)aminomethane (TRIS) HCL in water, set to a pH of 8.6, at 2 ml per sample at 37°C for 72 hours, refreshing the solution every 24 hours, rotating at 30 RPM with the samples being constantly submerged during rotation. The elastase solution was subsequently washed away by three times incubating with 10 ml deionized water for 5 min each rotating at 30 RPM at RT. To facilitate opening up of the lacunae and increase removal of cell debris out of the cartilage matrix, samples were exposed to physical strain: a snap freeze-thaw cycle (from -190°C to 37°C), sonication twice for 8 minutes, at 42 kHz (70 Watt waterbath sonicator) and vortexing thrice, in absence of liquid, for about 30 seconds at 2500 RPM.

Subsequently, sterility was tested and the samples coated with serum by incubating the samples in 5 ml MEM-α (Gibco, Carlsbad, USA), containing 10% heat-inactivated FCS (Lonza, Verviers, Belgium) for 24 hours at 37°C rotating at 30 RPM. Sterile samples were afterwards held in place using tweezers, and injected at three different sites, aiming for the centre most region, using a 30-gauge needle with about 1.500.000 cells, as detailed forth following, in 150 µl MEM-α (Gibco, Carlsbad, USA), containing 10% heat-inactivated FCS (Lonza, Verviers, Belgium). Cells were either human adult bone marrow-derived stromal cells (BMSCs) or human articular chondrocytes isolated from osteoarthritic knee joints of patients undergoing total knee replacement, expanded for 10 days in DMEM (Lonza) with 10% heat-inactivated FCS (Lonza), gentamicin (50 mg/mL; Invitrogen Life Technologies), and 1.5 mg/mL amphotericin B (Fungizone; Invitrogen Life Technologies). Following injection, samples were gently rinsed thrice in phosphate buffered saline and transferred to 10 cm² wells (each sample occupying one well) and cultured for 35 days with medium renewal every three days in 4 ml/well of chondrogenic medium (DMEM-high glucose GlutaMAX+ (GIBCO), 1:100 insulin, transferrin and selenous acid (ITS+, BD Biosciences), 40 µg/ml L-proline (Sigma-Aldrich), 1 mM sodium pyruvate (GIBCO), 100 nM dexamethasone (Sigma-Aldrich), 10 ng/ml transforming growth factor β1 (TGF-β1, R&D Systems), 1.5 µg/ml fungizone (Invitrogen Life Technologies) and 50 µg/ml gentamycin (Invitrogen Life Technologies)). However, any chondrogenic differentiation protocol should work, dependent on cell type and preference.

After culture, samples were harvested carefully with tweezers, washed gently with phosphate buffered saline (PBS) and fixed in 3.7% formalin for 24 hours. Fixed samples were then embedded in agarose, dehydrated, embedded in paraffin and sectioned at 6 µm thickness and the sections placed on glass slides and subsequently dried, rehydrated, stained with Haematoxylin (Sigma-Aldrich) and 2% Eosin (Merck) or 0.4% Thionin solution (Sigma-Aldrich), dehydrated, and sealed under a glass coverslip as described by described in Narcisi et al., 2015 (Narcisi et al., 2015. Stem Cell Reports 4: 459-7).

Micrographs (Figure 2) of the decellularized and reseeded samples show that cells have migrated from the injection site into the surrounding cartilage matrix and undergone chondrogenic differentiation, forming new cartilage-like matrix, which was visualized by staining for glycosaminoglycans using Thionin.

### Example 3: In vivo Chondrogenesis in joint model

To determine if elastase-treated elastic cartilage is suitable to repair cartilage defects, it was used to fill defects in a model of articular cartilage damage. First, the outer ears of 12-week-old calves were washed with water and the perichondrium, skin and muscle were removed using a scalpel till only the cartilage remained. Subsequently, biopsies of 6 mm diameter were obtained from the auricular cartilage using biopsy punches and cut to a height of either approximately 0.4 or 1.2 mm.

The protocol employed for removal of the cells and elastin fibers was identical to that described in example 1. The volumes given applied to batches of about 20 samples unless specified otherwise.

After removal of the cells and elastin fibers, half the samples were then placed at the bottom of a 0.3 cm² well (one sample/well, 96 well plate) and covered with 500.000 chondrogenic cells per sample as a solution in 200 µl of chondrocyte expansion medium (DMEM (Lonza) with 10% heat-inactivated FCS (Lonza), 50 mg/mL gentamicin (Invitrogen Life Technologies), and 1.5 mg/mL amphotericin B (Fungizone; Invitrogen Life Technologies)). Chondrogenic cells in this case were human articular chondrocytes isolated from osteoarthritic knee joints of patients undergoing total knee replacement and expanded for at least 10 days in chondrocyte expansion medium. The other half of samples was covered in cell-free chondrocyte expansion medium. Subsequently, samples were incubated statically for 24 hours at 37°C, with medium gently renewed after 8 hours. To induce chondrogenic differentiation, the samples were rinsed with PBS twice and cultured for 18 days with medium renewal every three days in 200 µl/well of chondrogenic medium (DMEM-high glucose GlutaMAX+ (GIBCO), 1:100 insulin, transferrin and selenous acid (ITS+, BD Biosciences), 40 µg/ml L-proline (Sigma-Aldrich), 1 mM sodium pyruvate (GIBCO), 100 nM dexamethasone (Sigma-Aldrich), 10 ng/ml transforming growth factor β1 (TGF-β1, R&D Systems), 1.5 µg/ml fungizone (Invitrogen Life Technologies) and 50 µg/ml gentamycin (Invitrogen Life Technologies).

For a model of articular cartilage damage, we took 8 mm diameter biopsies from the articular surface of the metacarpal-phalangeal joint of 12-week old calves using a diamond-coated trephine drill (Synthes) and biopsy punches as described by de Vries-van Melle et al., 2011 (de Vries-van Melle et al., 2011. Tissue Eng Part C Methods 18: 45-53). Subsequently, the articular bone-cartilage biopsies were cut to 4 mm in height and a 6 mm defect created either approximately 0.4 mm deep, affecting only the cartilage layer (chondral defect) or approximately 1 mm deep, into the bone (osteochondral defect). These articular cartilage and bone biopsies, hereon referred to as joint models, were then rinsed thoroughly in PBS, incubated statically for 24 hours at 37°C. The next day, the elastase-treated ear cartilage samples, either seeded with chondrocytes or unseeded, were placed in the joint defect models according to matching height.

The combined joint model with decellularized cartilage sample was then implanted subcutaneously into female NMRI nu/nu mice (Charles River, Wilmington, MA) under a Neuro-Patch membrane (Braun, Melsungen, Germany), to prevent invasion of murine skin cells, as described by de Vries-van Melle et al., 2014 (de Vries-van Melle et al., 2014. Eur Cell Mater 27: 112-23) and harvested after 2, 4, 8 or 10 weeks.

The samples were subsequently fixed using 4% formalin for 5 days at room temperature, and decalcified using 10% formic acid for at least 7 days, with refreshment of the solution every 3 days. The samples were then washed with deionized water until neutral pH and embedded in agarose, dehydrated, embedded in paraffin and sectioned at 6 µm thickness. The sections were placed on glass slides and subsequently dried, rehydrated, stained with Haematoxylin (Sigma-Aldrich) and 2% Eosin (Merck) or 0.4% Thionin solution (Sigma-Aldrich), dehydrated, and sealed under a glass coverslip as described by described in Narcisi et al., 2015 (Narcisi et al., 2015. Stem Cell Reports 4: 459-7).

Micrographs show that in the osteochondral defect, cells from the bony part of the bovine joint defect model and, to a minor extent, cells from the cartilage part of the bovine joint defect model, invade the non-seeded decellularized elastin-reduced elastic cartilage, forming cartilage (white arrowheads) from 2-4 weeks (Figure 3A,B).

In the chondral defects, shown in Figure 3C and D, invasion of cells from the bony part of the bovine joint defect model is prevented since the remaining layer of calcified cartilage (indicated by shaded arrowheads) blocks cell migration. Here only cells from the cartilage part of the bovine joint defect model invade the non-seeded decellularized elastin-reduced elastic cartilage and form new cartilage (white arrowheads) that remains stable at 10 weeks.

When elastin-reduced elastic cartilage was seeded with human articular chondrocytes prior to implantation, the seeded human articular chondrocytes appeared to prevent additional invasion of cells after implantation. Figure 3E and F show that an elastin-reduced elastic cartilage sample (dotted white outline) seeded with human articular chondrocytes showed local chondrogenesis (white arrowheads) and formation of cartilage-like matrix within a chondral defect.

## Claims

1. A method of producing an elastin-reduced cartilage scaffold comprising the steps of:
- providing an elastic cartilage sample,
- reducing elastin from said cartilage sample to produce an elastin-reduced cartilage scaffold.

2. The method according to claim 1, wherein the elastin is reduced to about 50%, preferably about 25%, preferably about 10%, preferably about 1%, by incubation with one or more enzymes, preferably comprising an elastase such as porcine pancreatic elastase.

3. The method according to claim 2, wherein the cartilage scaffold is incubated with 1-12 U/ml elastase, preferably about 3 U/ml.

4. The method according to claim 2, wherein the cartilage scaffold is incubated with 0.01-1 U/ml elastase in combination with at least one proteolytic enzyme, preferably pepsin.

5. The method according to any one of claims 1-4, wherein the cartilage scaffold is subjected to a decellularization step, prior to reduction of elastin.

6. The method according to any one of claims 1-5, wherein the cartilage scaffold is subjected to a decontamination step, prior to or after reduction of elastin.

7. The method according to claim 2, 3 or 4, wherein the incubation with elastase comprises incubation of the cartilage scaffold for a period of 1-80 hours, to reduce elastin.

8. An elastin-reduced cartilage scaffold obtainable by the method of any one of claims 1-7.

9. The method according to any one of claims 1-7, further comprising recellularizing the elastin-reduced cartilage scaffold by adhering cells to said scaffold.

10. The method according to claim 9, wherein the elastin-reduced cartilage scaffold is recellularized by migration of cells into the elastin-reduced cartilage scaffold, preferably by seeding cells onto the cartilage scaffold or by injecting cells into the cartilage scaffold.

11. The method according to claim 9 or claim 10, wherein the cells used for recellularization of the cartilage scaffold are cells with chondrogenic or osteogenic potential such as chondrocytes, perichondrium cells, periosteum cells, and stem/progenitor cells, such as adipose-derived stromal vascular stem cells, synovium-derived mesenchymal cells and bone marrow-derived stromal cells.

12. The method according to any one of claims 9-11, wherein the cells used for recellularization of the cartilage scaffold are autologous human cells, obtained from a recipient of said cartilage scaffold.

13. The method according to any one of claims 9-11, wherein the cells used for recellularization of the cartilage scaffold are allogeneic human cells, obtained from an individual that is not a recipient of said cartilage scaffold.

14. A recellularized elastin-reduced cartilage scaffold obtainable by the method of any of claims 9-13.

15. The cartilage scaffold according to claim 8 or 14, for use in a method of repairment of cartilage, such as articular, nasal, airway and ear cartilage, repairment of an osteochondral defect and repairment of a bone defect.
